# EUROPEAN PATENT APPLICATION

(11) **EP 0 979 644 A1**
(43) Date of publication of application: **16.02.2000**
(21) Application number: 99830439.8
(22) Date of filing: 08.07.1999
(51) Int. Cl.: A61K 7/32

(54) **Deodorant and/or moisturizing cosmetic composition**

(30) Priority: 13.08.1998 IT MI981895
(71) Applicant: Società Italo-Britannica L. Manetti-H. Roberts & C. S.p.A., 50123 Firenze (IT)
(72) Inventor: Mucci, Paolo, 50100 Firenze (IT); Meucci, Sandro, 50100 Firenze (IT); Ceccarelli, Luigi, 25036 Palazzolo Sull'Oglio, (Brescia) (IT)
(74) Representative: Bardini, Marco Luigi

(57) **Abstract**

A deodorant and/or moisturizing cosmetic composition formed by an emulsion oil in water, comprising: a) a combination of two or more deodorant and/or moisturizing active ingredients, b) an orthophosphonic acid ester in an amount comprised between 0.3 and 7%, and c) a watersoluble resin consisting of a homopolymer of the acrylic acid in an amount comprised between 0.05% and 1.5%, said composition having a viscosity at 20°C comprised between 800 and 10000 caps. The main advantage of the cosmetic composition according to the invention consists in that it is a cream and, at the same time, is vaporizable, as the viscosity is always comprised in the above stated range.

## Description

The present invention relates to a deodorant and/or moisturizing cosmetic composition.

The problem of the deodoration of human body has been tackled in several ways with products of different forms and compositions. In particular, the deodorant product may be made available in the solid form, as a cream or as a liquid. Different ways of application also correspond to the different forms. The stick is used for products available in the solid form, while the roll-on or the direct product massaging are the most common application modes when the product is available as a cream. Aerosol, vaporizers or spray dispensers are used when the products are in the liquid form.

The vaporizable liquid deodorants are comprised within the wide group of cosmetic deodorants and comprise commercially available preparations containing antiperspirant substances, other active ingredients, fragrance, essential oils, etc. with or without alcohol.

Vaporizable liquid deodorants are also known containing emollient substances of various origin, to improve the moisturizing properties of the product and make it specially delicate and tolerable for the skin. The viscosity of these products is similar to that of water.

Deodorants products defined as deodorant cleansing lotion or deodorant cream are also commercially available in the form of a liquid emulsion. They have a milky white color which could give the impression that they are softer and more delicate products than the transparent liquid products, but they have in any case a liquid consistency: therefore, particularly for this type of liquid product, which requires some time to dry, the inconvenience has been found that it can trickle down the body.

On the other hand, the deodorants in the form of a cream, which have a viscosity high enough that the use of a tube container or the roll-on is allowed, are applied in the above mentioned ways, i.e. through massaging in the case of the cream contained in a tube or directly by means of the roll-on.

Furthermore, due to the higher viscosity of the cream product, it may happen that the body part, which the product has been applied on, remains tacky and slightly oily, with an unpleasant feeling for the user and problems, for example, of soiling the clothes; it may be also necessary to wash the hands after the product has been applied.

The features of the prior art products and compositions do not allow for a deodorant and/or moisturizing product to be obtained as a stable cream suitable of being vaporized. It is therefore an object of the present invention to provide a deodorant and/or moisturizing product having a cream consistency and, at the same time, suitable of being vaporized, thus overcoming the above mentioned prior art incoveniences.

The above result has been achieved by the deodorant and/or moisturizing cosmetic composition according to the invention consisting by an oil-in-water emulsion, characterized in that it comprises:
a) a combination of two or more deodorant and/or moisturizing active ingredients,
b) an ester of the ortophosphoric acid in an amount comprised between 0.3% and 7%,
c) a water-soluble resin consisting of an acrylic acid homopolymer in an amount comprised between 0.05% and 1.5%,
and further characterized in that the viscosity at 20°C is comprised between 800 and 10000 cps.

The deodorant and/or moisturizing cosmetic composition according to the invention consists, therefore, of a deodorant and/or moisturizing product in the form of a fluid cream of medium/low viscosity, capable of being vaporized.

The essential advantage of the deodorant and/or moisturizing cosmetic composition according to the present invention consists in that it is costantly in the form of a cream and, at the same time, is vaporizable, as its viscosity at 20°C is always comprised in the above mentioned range and, even if small variations may occur, it never falls below 800 cps nor goes beyond 10000 cps at least during the first three months.

A further object of the invention is the use of the above mentioned cosmetic composition as a body deodorant and/or moisturizing product.

In particular, the deodorant and/or moisturizing cosmetic composition according to the invention can be used, thanks to its features, as a deodorant and/or moisturizing product for armpits, feet and other body parts requiring deodorizing and/or moisturizing.

The oil-in-water emulsion, which is the basis of the deodorant and/or moisturizing cosmetic composition according to the present invention, can be used also as a basis for creamy and vaporizable deodorant and/or moisturizing cosmetic compositions, containing further active ingredients such as emollients, preservatives, antioxidants, moisturizing/humectant substances and/or perfume.

Such moisturizing components allow to obtain a product in which the moisturizing property is enhanced for skins with increased need in this respect.

Moreover, the cosmetic composition as a vaporizable cream according to the invention can be advantageously used for further applications, such as, for example, a balsam, an emollient product, etc., if the combination of two or more deodorant and/or moisturizing active ingredients is partially or completely replaced by other active ingredients suitably selected according to the product to be obtained.

All the percentages given in the present specification have to be construed as weight percentages with respect to the total weight of the composition, unless otherwise specified.

The combination of deodorant and/or moisturizing active ingredients of the deodorant and/or moisturizing cosmetic composition of the present invention can be any combination of the following compounds: vitamin E acetate (toco-pheril acetate), farnesol (3,7, 11-trimethyl-2,6, 10-dodecatrien-1-ol), triethyl citrate, C₁₂-C₁₃ alkyl-lactate or C₁₂-C₁₃ alkyl citrate, denatured ethyl alcohol, triclosan (2,4,4'-trichloro-2'-hydroxydiphenil ether) L-arginine or arginine-PCA as deodorant active ingredients, and/or sodium pyroglutamate, sodium lactate, proteic hydrolysates (collagenic or cheratinic), reconstituted aminoacid mixtures, saccharidic components (glucose, fructose, mucopolysaccharides, etc.), polyalcohols (glycerole, sorbitole) and glycols (polyethylenglycol), as moisturizing active ingredients.

The combination of the deodorant and/or moisturizing active ingredients can be a combination of C₁₂-C₁₃ alkyl lactate (or C₁₂-C₁₃ alkyl citrate) in an amount not higher than 4%, Farnesol in an amount not higher than 1.5% and vitamin E acetate in an amount not higher than 1.5%. The combination may also comprise triethyl citrate in an amount not higher than 4% and/or denatured ethyl alcohol in an amount not higher than 10%.

The ester of the ortophosphoric acid is preferably a mixture of mono-, bi- and triesters of the ortophosphoric acid with alkyl tetraglycolethers, where the alkyl group is mainly a mixture of C₁₂ and C₁₄.

The ester of the ortophosphoric acid can be the trileuril (4) OE phosphate.

These products can be used at room temperature and exhibit an high emulsifying activity, mainly in the presence of paraffin oils and/or synthetic oils.

A commercial product known as Hostapath KL 340 N, corresponding to the trilauril (4) OE phosphate, is preferably used as ester of the ortophosphoric acid. The water-soluble resin of the deodorant and/or moisturizing cosmetic composition according to the present invention, consisting of an acrylic acid homopolymer, i.e. carboxypolymethylene, is commercially available under the name Carbopol 5/984.

The acrylic acid homopolymers exhibit a viscosity-increasing and stabilizing action on the emulsions: this component is used not only to make the emulsion thicker, but also in view of its ability to impart a slipping coefficient to the emulsion.

The presence of both the orthophosphoric acid ester and the water-soluble resin is essential for the preparation of a deodorant and/or moisturizing cosmetic composition capable of being easily pumped, with a low resistance to handling.

As set forth previously, the deodorant and/or moisturizing cosmetic composition according to the present invention comprises a combination of two or more deodorant and/or moisturizing active ingredients.

In particular, vitamin E acetate can be present in an amount comprised between O and 1.5% approx. and is added to the deodorant and/or moisturizing cosmetic composition according to the present invention even for its antioxidant properties. Vitamin E protects the skin by preventing free-radicals formation and, at the same time, maintains it moist, in view of the fact that reduces the loss of water.

Farnesol (3,7, il-trimethyl-2,6,10-dodecontrien-1-ol) is a natural-like bacteriostatic agent and can be added in an amount comprised between about 0 and 1.5% and exhibits an inhybitory action against bacterial growth and degradation, which are responsible for the formation of malodour. It can be found in the essential oils of many natural products.

Triethyl citrate can be present alone or in combination with buthyl-hydroxy-toluene (BHT), an antioxidant agent, in an amount comprised between about 0 and 4%. Triethyl citrate exhibits a deodorizing action as prevents the enzymatic decomposition of the fatty acids contained in the sweat, due to microorganisms usually present on the skin. In this way, malodorous degradation products do not form and the physiologic perspiration process is not altered.

BHT, which, as said above, is an antioxidant, acts synergistically with triethyl citrate to prevent the decomposition of some insaturated fatty acids of the skin into malodorous substances.

The triethyl citrate to be used is preferably the commercial product named Citrogen-Deo.

BHT can be present in the composition according to the present invention in an amount comprised between about 0 and 1%.

As already stated, another possible deodorant active ingredient is C₁₂-C₁₃-alkyl lactate which is added in an amount comprised between about 0 and 4%. This ingredient is very important for its considerable, marked deodorant properties, having also a slightly bacteriostatic effect; such ingredient also develops a dermoprotective action if it is associated to antibacterial agents, thanks to its emollient and moisturizing properties. The agent is commercially available under the name Cosmacol ELI. Furthermore, being a lactic acid carrier, it has many properties of the fruit acids, but differently to them, its action is extremely soft, i.e. without unwanted side effects. Therefore, it can be used even in products for the most delicate skins.

In the place of, or in addition to, C₁₂-C₁₃ alkyl lactate there can be used another deodorant/emollient active ingredient of the same family of the previous one, i.e. the C₁₂-C₁₃ alkyl citrate, a triester of the citric acid with the C₁₂-C₁₃ monobranched alcohol. This ester is a citric acid carrier, an α-hydroxyacid (AHA) with particular emollient and antioxidant properties, and is known commercially as Cosmacol ECI and can be added in an amount comprised between 0 and 1%.

The denatured ethyl alcohol can be added to an amount comprised between 0 and 10% and increases the feel of freshness, enhancing at the same time the bacteriostatic action of the composition.

A further active ingredient which can be used in the composition according to the invention is the triclosan (2,4,4'-trichloro-2'-hydroxydiphenil ether) . Triclosan is a wide-spectrum antibacterial agent which is active against bacteria (Gram+, Gram-, leavens and moulds) responsible for sweat degradation and therefore for the malodour formation. It exhibits a good skin tolerability and is compatible with the other ingredients. It is commercially available under the name of DP300 and can be added in an amount up to 0.3%.

L-arginine and arginine-PCA (a compound of arginine and pyrrolidon carboxylic acid exhibit deodorant properties thanks to their antioxidant activity like vitamin E. They also exhibit moisturizing properties because they can retain water on the skin surface.

The above mentioned moisturizing active ingredients prevent skin dehydratation phenomena from one side and restore, at least at the corneous level, the normal conditions of hydratation, softness and elasticity from the other side.

The moisturizing and deodorant cosmetic composition according to the invention may contain preferably sodium pyroglutamate in an amoount comprised between 0 and 5%.

Sodium pyroglutamate is a substance contained in a large amount in the corneous layer of the skin (it is one of the components of the natural moisturizing factor - NMF) and is used as moisturizing active ingredient in view of its high hygroscopicity which increases the water content of the skin and gives elasticity and softness to it. This substance is commercially available under the name Adjdew NL-50.

The composition according to the present invention may also contain propylene glycol as moisturizing/humectant active ingredient in an amount comprised between about 0 and 7%.

As previously stated, the deodorant and moisturizing composition according to the present invention may comprise other components.

For example, it may comprise an emollient ingredient which can be selected from triglycerides of fatty acids of natural origin and vaselin oil.

In particular, among the emollient ingredients particularly preferred is a triglyceride of fatty acids of natural origin, i.e. the caprilic/capric triglyceride, commercially known as Myritol 812, in an amount comprised between about 3 and 7%.

It exhibits a good dermatological compatibility and a good solubilizing power against liposoluble additives.

The vaseline oil may be present in an amount comprised between 5 and 10%.

The deodorant and moisturizing cosmetic composition according to the present invention may also contain a preservative preferably consisting of a mixture of diazolidinil urea and paraben (parahydroxybenzoic acid esters).

The commercial name of the preferred preservative according to the present invention is Germaben II. It consists of a mixture of propylene glycol, diazolidinil urea, methylparaben and propylparaben and is present in an amoount comprised between 0 and 2%. The deodorant and moisturizing cosmetic composition according to the present invention may also contain an antioxidant which may be butyl-hydroxy-toluene (BHT) and/or sodium EDTA.

Sodium hydroxyde is used to neutralize the homopolymer of the acrylic acid and is added in an amount proportional to it.

Disodium EDTA is used as complexing agent of the calcium and magnesium salts present in water, when not fully deionized water is used. The deodorant and/or moisturizing cosmetic composition according to the invention can also contain a perfume in an amount comprised between about 0 and 5%.

The main advantage of the deodorant and/or moisturizing cosmetic composition according to the present invention consists in that it is an emulsion with the appearance of a, more or less viscous, but not liquid, fluid cream, having a deodorant and/or moisturizing effect, capable of being vaporized by means of a micropump. Therefore, the product has, at the same time, the advantage of the cream product (for example, it does not trickle down the armpits after its application, before drying) and of the vaporizable product (there is no need to spread the product with the fingers or by means of the roll-on). Therefore, the application of the product is extremely practical and pleasant for the user, because it is fluid enough to be capable of being vaporized and at the same time viscous enough not to trickle down the armpits and the body.

A further advantage of the cosmetic composition according to the present invention is to be a viscous cream exhibiting at the same time moisturizing, emollient and deodorant action.

The features and advantages of the composition according to the invention will be better understood from the following detailed exemplifying description.

### Example 1

### Method for preparing a deodorant and/or moisturizing cosmetic composition in the form of a vaporizable cream

The deodorant and/or moisturizing cosmetic composition according to the present invention is prepared as follows.

Vaseline oil, Myritol 312, Hostapath KL 340 N, Cosmacol ELI, Farnesol, triethyl citrate, perfume, vitamin E acetate, Germaben II and BHT (i.e. the selected deodorant active ingredients, the ester of the ortophosphoric acid, an antioxidant, an emollient, a preservative and perfume) were introduced in a stainless steel reactor. The mixture was stirred until a complete dissolution is reached.

Water, propylene glycol, disodium EDTA, Carbopol 5/984 (i.e. water, the water-soluble resin, a humectant agent and an antioxidant) were introduced in a second stainless steel reactor equipped with an homogenizer and the mixture was homogenized up to a complete dispersion of the Carbopol 5/984.

The two mixtures obtained in this way were then combined together. Sodium hydroxyde, which was previously solubilized in water, was added under slow stirring and the resulting mixture was stirred and homogenized until a fluid and smooth emulsion is obtained.

If moisturizing active ingredients, such as sodium pyroglutamate, are present, these are added preferably at the end of the above described operation. Then, once the fluid and smooth emulsion is obtained, the moisturizing agent is added and the mixture is stirred up to dissolution.

The formulation prepared in this way has the following specifications:
Appearance: white homogeneous emulsion;
Density (20°C): 0.980 - 0.990;
Viscosity (20°C): 800-10000 cps (Brookfield-TB wheel - 5rpm) measured after 18 hours;
Reaction: pH 5.0 - 7.0;
Stability: no separation occurs;
Total microbial counting: lower than 100 UFC/ml.

### Example 2

A deodorant and/or moisturizing cosmetic composition in the form of a vaporizable cream was prepared as described in example 1, with the components listed in the following table:

| Components | Weight % |
|---|---|
| Water | 74,876 |
| Vaseline Oil | 8,000 |
| Propylene Glycol | 5,000 |
| Myritol 312 | 5,000 |
| Hostaphat KL 340 N | 3,000 |
| Cosmacol ELI | 1,000 |
| Perfume | 1,000 |
| Farnesol | 0,600 |
| Vitamin E acetate | 0,500 |
| Germaben II | 0,500 |
| Carbopol 5/984 | 0,300 |
| Sodium Hydroxide | 0,124 |
| Bisodium Salt EDTA | 0,050 |
| BHT | 0,050 |

The viscosity at 20°C (initial) ranges from 2000 cps to 3000 cps.

### Example 3

A deodorant and/or moisturizing cosmetic composition in the form of a vaporizable cream was prepared as described in example 1, with the components listed in the following table:

| Components | Weight % |
|---|---|
| Water | 72,170 |
| Vaseline Oil | 8,000 |
| Propylene Glycol | 5,000 |
| Myritol 312 | 5,000 |
| Hostaphat KL 340 N | 3,000 |
| Triethyl citrate | 2,000 |
| Cosmacol ELI | 1,000 |
| Perfume | 1,000 |
| Farnesol | 0,600 |
| Vitamin E acetate | 0,500 |
| Germaben II | 0,500 |
| Carbopol 5/984 | 0,800 |
| Sodium Hydroxide | 0,330 |
| Bisodium Salt EDTA | 0,050 |
| BHT | 0,050 |

The viscosity at 20°C (initial) ranges from 4000 cpc to 6000 cpc.

### Example 4

A deodorant and/or moisturizing cosmetic composition in the form of a vaporizable cream was prepared as described in example 1, with the components listed in the following table:

| Components | Weight % |
|---|---|
| Water | 73,733 |
| Vaseline Oil | 8,000 |
| Propylene Glycol | 5,000 |
| Myritol 312 | 5,000 |
| Hostaphat KL 340 N | 3,000 |
| Cosmacol ELI | 2,000 |
| Perfume | 1,000 |
| Farnesol | 0,600 |
| Vitamin E acetate | 0,500 |
| Germaben II | 0,500 |
| Carbopol 5/984 | 0,330 |
| Sodium Hydroxide | 0,137 |
| Triethyl citrate | 0,100 |
| Bisodium Salt EDTA | 0,050 |
| BHT | 0,050 |

The viscosity at 20°C (initial) ranges from 2000 cps to 3000 cps.

### Example 5

A deodorant and/or moisturizing cosmetic composition in the form of a vaporizable cream was prepared as described in example 1, with the components listed in the following table:

| Components | Weight % |
|---|---|
| Water | 72,733 |
| Vaseline Oil | 8,000 |
| Propylene Glycol | 5,000 |
| Myritol 312 | 5,000 |
| Hostaphat KL 340 N | 3,000 |
| Cosmacol ELI | 2,000 |
| Denatured ethyl alcohol | 1,000 |
| Perfume | 1,000 |
| Farnesol | 0,600 |
| Vitamin E acetate | 0,500 |
| Germaben II | 0,500 |
| Carbopol 5/984 | 0,330 |
| Sodium Hydroxide | 0,137 |
| Triethyl citrate | 0,100 |
| Bisodium Salt EDTA | 0,050 |
| BHT | 0,050 |

The viscosity at 20°C (initial) ranges from 2000 cps to 3000 cps.

### Example 6

A deodorant and/or moisturizing cosmetic composition in the form of a vaporizable cream was prepared as described in example 1, with the components listed in the following table:

| Components | Weight % |
|---|---|
| Water | 74,433 |
| Vaseline Oil | 8,000 |
| Propylene Glycol | 5,000 |
| Myritol 312 | 5,000 |
| Hostaphat KL 340 N | 3,000 |
| Adjdew NL-50 | 2,000 |
| Perfume | 1,000 |
| Vitamin E acetate | 0,500 |
| Germaben II | 0,500 |
| Carbopol 5/984 | 0,330 |
| Sodium Hydroxide | 0,137 |
| Bisodium Salt EDTA | 0,050 |
| BHT | 0,050 |

The viscosity at 20°C (initial) ranges from 2000 cps to 3000 cps.

### Example 7

A deodorant/moisturizing cosmetic composition in the form of a vaporizable cream is prepared as described in example 1 with the components listed in the following table:

| Components | Weight % |
|---|---|
| Water | 75,276 |
| Vaseline Oil | 8,000 |
| Propylene Glycol | 5,000 |
| Myritol 312 | 5,000 |
| Hostaphat KL 340 N | 3,000 |
| Cosmecol ECI | 1,000 |
| Perfume | 1,000 |
| Vitamin E acetate | 0,500 |
| Germaben II | 0,500 |
| Carbopol 5/984 | 0,300 |
| Irgosan DP 300 | 0,200 |
| Sodium Hydroxide | 0,124 |
| Bisodium Salt EDTA | 0,050 |
| BHT | 0,050 |

The viscosity at 20°C (initial) ranges from 2000 cps to 3000 cps.

## Claims

1. Deodorant and/or moisturizing cosmetic composition consisting of an emulsion of oil in water, characterized in that it comprises:
- a combination of at least two deodorant and/or moisturizing active ingredients;
- an ortophosphoric acid ester in an amount comprised between 0.3% and 7%;
- a water-soluble resin consisting of an homopolymer of the acrylic acid in an amount comprised between 0.05% and 1.5%,
and further characterized in that the viscosity at 20°C is comprised between 800 and 10000 cps.

2. Deodorant and/or moisturizing cosmetic composition according to claim 1, characterized in that it is in the form of a vaporizable cream.

3. Deodorant and/or moisturizing cosmetic composition according to claim 1, characterized in that it also comprises emollient, preservative, antioxidant, moisturizing/humectant ingredients and/or perfume.

4. Deodorant and/or moisturizing cosmetic composition according to claim 1, characterized in that the combination of the deodorant and/or moisturizing active ingredients is any combination of the following substances: vitamine E acetate (tocopheryl acetate), Farnesol (3,7,11-trimethyl-2,6,10-dodecatrien-1-ol), triethyl citrate, C₁₂-C₁₃ alkyl lactate, C₁₂-C₁₃ alkyl citrate, denatured ethyl alcohol, triclosan (2,4,4'-trichloro-2'-hydroxydiphenil ether), L-arginine and arginine-PCA as deodorant active ingredients, and/or sodium pyroglutamate, sodium lactate, proteic hydrolysates, reconstituted mixtures of aminoacids, saccharidic components, polyalcohols and glycols.

5. Deodorant and/or moisturizing cosmetic composition according to claim 1, characterized in that the combination of the deodorant active ingredients is a combination of C₁₂-C₁₃ alkyl lactate in an amount not greater than 4%, Farnesol in an amount not greater than 1.5% and vitamin E acetate in an amount not greater than 1.5%.

6. Deodorant and/or moisturizing cosmetic composition according to claim 5, characterized in that it also comprises triethyl citrate in an amoount not greater than 4%.

7. Deodorant and/or moisturizing cosmetic composition according to claim 5, characterized in that it also comprises denatured ethyl alcohol in an amoount not greater than 10%.

8. Deodorant and/or moisturizing cosmetic composition according to claim 5, characterized in that it also comprises triethyl citrate in an amount not greater than 4% and denatured ethyl alcohol in an amount not greater than 10%.

9. Deodorant and/or moisturizing cosmetic composition according to claim 1, characterized in that the ester of the orthophosphoric acid is a mixture of mono-, di- and tri-esters of the ortophosphoric acid with alkyl tetraglycol ethers, wherein the alkyl group is mainly a mixture of C₁₂-C₁₄.

10. Deodorant and/or moisturizing cosmetic composition according to claim 1, characterized in that the ester of the ortophosphoric acid is trilauril(4)OE phosphate.

11. Deodorant and/or moisturizing cosmetic composition according to claim 1, characterized in that the acrylic acid homopolymer is carboxypolymethylene (Carbopol 5/984).

12. Deodorant and/or moisturizing cosmetic composition according to claim 3, characterized in that the emollient active ingredient is selected from natural fatty acid triglycerides and vaseline oil.

13. Deodorant and/or moisturizing cosmetic composition according to claim 3, characterized in that the emollient active ingredient is the caprilic/capric triglyceride, in an amount comprised between 3 and 7%.

14. Deodorant and/or moisturizing cosmetic composition according to claim 3, characterized in that the preservative consists of a mixture of diazolidinil urea and parabens.

15. Deodorant and/or moisturizing cosmetic composition according to claim 3, characterized in that the preservative consists of a mixture of propylene glycol, diazolidinil urea, methylparaben and propylparaben in an amount comprised between 0 and 2%.

16. Deodorant and/or moisturizing cosmetic composition according to claim 3, characterized in that the antioxidant can be buthyl-hydroxy-toluene (BHT), sodium hydroxyde, and/or bisodium EDTA.

17. Deodorant and/or moisturizing cosmetic composition according to claim 3, characterized in that it comprises triethyl citrate as deodorant active ingredient and buthyl-hydroxy-toluene as antioxidant.

18. Deodorant and/or moisturizing cosmetic composition according to claim 4, characterized in that the combination of deodorant and/or moisturizing active ingredients comprises sodium pyroglutamate in an amount not greater than 5%.

19. Deodorant and/or moisturizing cosmetic composition according to claim 3, characterized in that the humectant agent is propylene glycol in an amount comprised between 0 and 7% approximately.

20. Deodorant and/or moisturizing cosmetic composition according to claim 3, characterized in that it comprises a perfume in an amount comprised between 0 and 5%.

21. Deodorant and/or moisturizing cosmetic composition according to claim 3, characterized in that it comprises C₁₂-C₁₃ alkyl lactate in an amount of 1%, Farnesol in an amount of 0.6%, trilauril (4) OE phosphate in an amount of 3%, vitamin E acetate in an amount of 0.5% and carboxypolymethylene (Carbopol 5/984) in an amount of 0.3%.

22. Deodorant and/or moisturizing cosmetic composition according to claim 1, characterized in that the combination of deodorant active ingredients is a combination of C₁₂-C₁₃ alkyl citrate, in amount not higher than 4%, vitamin E acetate in an amount not higher than 1.5% and triclosan in an amount not higher than 0.3%.

23. A deodorant and/or moisturizing cream having the composition according to any one of the claims 1-22.

24. Use of the deodorant and/or moisturizing cosmetic composition according to any one of the claims 1-22, as a deodorant and/or moisturizing body product.

25. The use according to claim 24, as a deodorant and/or moisturizing product for armpits, feet or other body parts requiring deodorizing or moisturizing.
